Europäisches Patentamt

European Patent Office

Office européen des brevets

(11) Veröffentlichungsnummer: **0 211 822**
**A2**

(12) **EUROPÄISCHE PATENTANMELDUNG**

(21) Anmeldenummer: **86890210.7**

(22) Anmeldetag: **15.07.86**

(51) Int. Cl.⁴: **A 61 N 1/32**

(30) Priorität: **31.07.85 AT 2257/85**

(43) Veröffentlichungstag der Anmeldung:
**25.02.87 Patentblatt 87/9**

(84) Benannte Vertragsstaaten:
**AT BE CH DE FR GB IT LI LU NL SE**

(71) Anmelder: **HEPAX Limited**
**Meierhofstrasse 325**
**FL-9495 Triesen(LI)**

(72) Erfinder: **Dietheim, Eugen Franz**
**Meierhofstrasse 325**
**FL-9495 Triesen(LI)**

(74) Vertreter: **Krause, Walter, Dr. Dipl.-Ing.**
**Postfach 200 Singerstrasse 8**
**A-1014 Wien(AT)**

(54) **Elektromedizinisches Therapiegerät.**

(57) Die herkömmliche zumeist medikamentöse Behandlung von Herpes-Erkrankungen ist zeitaufwendig, großteils nur symptomatisch und vor allem bei rezidivierenden Formen nicht zufriedenstellend. Zur Beseitigung dieser Nachteile sieht die Erfindung ein elektromedizinisches Therapiegerät vor, mit welchem der Hautoberfläche eines Patienten monopolare Stromimpulse zuführbar sind, wobei die Frequenz der Stromimpulse zwischen 25 und 35 Hz liegt und die Impulsdauer zwischen 0, 1 und 0, 3 ms beträgt, sowie daß die Stromstärke abhängig von den über eine Eingabeeinrichtung (21) vorgebbaren Werten kleiner gleich 60 mA beträgt, wodurch Herpesviren in organischem Gewebe inaktiviert werden.

EP 0 211 822 A2

./...

_Fig.1_

0211822

Elektromedizinisches Therapiegerät

Die Erfindung betrifft ein elektromedizinisches Therapiegerät, mit einer Einheit zur Konstantstromerzeugung, welches eine Folge von monopolaren Stromimpulsen von annähernd Rechteckform erzeugt, die über zwei an der Sekundärwicklung eines Impulstransformators angeschlossene Elektroden der Hautoberfläche eines Patienten zuführbar sind.

Aus der DE-OS 1 489 686 ist ein Therapiegerät der eingangs genannten Art bekannt, bei welchem zum Zwecke der Schlaftherapie einem Patienten Rechteckimpulse einer bestimmten Frequenz, Impulsdauer und Intensität zugeführt werden. Die brauchbare Frequenz dieser Rechteckimpulse liegt dabei erfahrungsgemäß zwischen 5 und 100 Hz, während die Impulslänge 0,4 bis 2 ms betragen soll. Dabei zeigt es sich, daß es bei der Schlaftherapie unter anderem darauf ankommt, hinsichtlich Frequenz, Impulsdauer und Intensität der Impulse eindeutige und gleichbleibende Verhältnisse zu schaffen, wobei jede Rückwirkung auf die Rechteckimpulse aus dem Elektrodenkreis möglichst ausgeschlossen werden soll. In der genannten DE-OS werden Maßnahmen vorgeschlagen, wodurch eine Einheit zur Konstantstromerzeugung realisiert wird.

Der Erfindung liegt die Beobachtung zugrunde, daß Herpesviren elektrotherapeutisch inaktiviert werden können. Aufgabe der vorliegenden Erfindung ist es, ein Reizstromgerät der eingangs genannten Art so weiterzubilden, daß seine Anwendung zur gewünschten inaktivierenden Wirkung auf Herpesviren in organischem Gewebe führt.

Dieser Gedanke wird erfindungsgemäß dadurch realisiert, daß die Frequenz der Stromimpulse zwischen 25 und 35 Hz liegt und die Impulsdauer zwischen 0,1 und 0,3 ms beträgt, sowie daß die Stromstärke abhängig von den über eine Eingabeeinrichtung vorgebbaren Werten $\leq$ 60 mA beträgt, wodurch Herpesviren in organischem Gewebe inaktiviert werden. Erst durch die überraschend gefundene erfindungsgemäße Impulsform war es möglich, ein Therapiegerät zu schaffen, mit welchem bei einer relativ kurzen Behandlungsdauer - verglichen mit herkömmlichen ausschließlich medikamentösen Behandlungsformen - die Heilung herpaler Erkrankungen möglich ist.

Im folgenden werden, wenn nicht näher bezeichnet, unter Herpes alle Viruserkrankungen der Haut und Schleimhaut zusammengefaßt, welche auf die Infektion mit dem Herpes-simplex-Virus (Herpes-simplex) und auf Infektion mit dem Varizellenvirus (Herpes-zoster) zurückzuführen sind.

Die gängige medikamentöse Behandlung von Herpes-simplex ist großteils nur symptomatisch, meist langwierig und vor allem bei rezidivierenden Formen nicht zufriedenstellen. Zum Einsatz kommen unter anderem Virostatika in Lösungs- und Salbenform, deren Anwendung über den Zeitraum von einigen Wochen empfohlen wird. Außerdem bieten sich austrocknende und desinfizierende Maßnahmen zur Behandlung der betroffenen Hautstellen an.

Es ist auch keine befriedigende Therapie des Herpes-zoster bekannt. Es werden zwar die Schmerzen symptomatisch mit Analgetika behandelt, der Erfolg jeder ursächlichen Therapie ist jedoch umstritten. Ein großes Problem ist die Behandlung der Neuralgien, wobei auch Psychopharmaka und Tranquilizer verordnet werden, sowie in Extremfällen die durchtrennung der betroffenen Nervenbahnen empfohlen wird.

Das erfindungsgemäße Therapiegerät eignet sich für die Behandlung aller Herpes-Formen, wobei bei minimaler physischer und psychischer Belastung des Patienten der Heilungsprozeß wesentlich beschleunigt und somit ein allenfalls notwendiger Krankenhausaufenthalt wesentlich verkürzt wird. Außerdem wird das Auftreten von Rezidiven wirksam zurückgedrängt.

So ist der Patient nach durchschnittlich acht Tagen ohne Schmerzen, bereits nach drei Tagen tritt eine deutliche Schmerzverringerung ein. Schon nach zwei Tagen tritt eine Verbesserung des Hautzustandes ein, d.h. die seziernierenden Hautstellen trocknen ab, der Patient braucht keine lokale Therapie mehr. Bei der herkömmlichen Behandlung, die praktisch ausschließlich invasiv ist, kommt es zu Behandlungszeiten bis zu 6 Monaten bei hospitalisierten Patienten. Es wird kein Einfluß auf Rezidive festgestellt. Neben unabschätzbaren Spätfolgen der angewandten Arzneimittel kommt es auch teilweise zu Unverträglichkeiten. Aufgrund der kompakten handlichen Form des Gerätes können Patienten mit leichteren Herpes-Formen ambulant oder auch zu Hause mittels Leihgeräten behandelt werden. Die postherpetischen Schmerzen nach der klassischen Herpes-Behandlung bringen den Patienten oft zum Neurochirurgen.
Auch bei diesen Patienten empfiehlt es sich, sie mit dem Therapiegerät nach der Erfindung zu behandeln, wobei die Schmerzen sehr stark sinken oder ganz verschwinden sollten, sodaß kein chirurgischer Eingriff notwendig wird. Bei herkömmlicher Behandlung ist oft eine Risotomie notwendig, wobei erfahrungsgemäß bei etwa der Hälfte der Patienten der Schmerz weiter bleibt.

Mit dem aus der eingangs genannten DE-OS bekannten Reizstromgerät wäre jedenfalls eine erfolgreiche Herpestherapie aus mehreren Gründen nicht möglich. So wird hier die Frequenz der Impulse zwischen 4 und 50 Hz variiert, während bei der vorliegenden Erfindung für die Dauer der Behandlung ein fester Frequenzwert vorliegt, welcher zwischen 25 und 35 Hz liegt. Ebenso eignet sich die bei der Schlaftherapie bevorzugt angewandte Impulsdauer von etwa 1,3 ms, wie in Versuchen bereits eindeutig festgestellt wurde, nicht für die Herpestherapie. Schließlich reicht auch

- 4 -

die an den Klemmen für die Elektroden abnehmbare Impulsspannung von 18 bis 25 Volt nicht aus, um die für die Behandlung herpaler Erkrankungen notwendigen Stromstärken zu erzielen.

Das erfindungsgemäße Gerät wird beispielsweise folgendermaßen angewandt:

a) es wird das dem jeweiligen Ort des Herpes-Befalls an der Körperoberfläche zugeordnete Dermatom ermittelt;

b) es werden die zwei Elektroden des Therapiegerätes leitend an der Hautoberfläche plaziert - eine davon als Anode direkt über dem zugehörigen Nerv und die andere als Kathode an möglichst weit von der Anode entfernter Stelle desselben Dermatoms;

c) über die Elektroden wird Strom in Form von monopolaren und zumindest annähernd rechteckförmigen Impulsen zugeführt, wobei die Stromstärke in Abhängigkeit des jeweiligen Hautwiderstandes geregelt wird.

Damit steht erstmals ein Therapiegerät zur nichtinvasiven Behandlung für alle Formen des Herpes-simplex und des Herpes-zoster zur Verfügung. Die mit dem erfindungsgemäßen Gerät mit seiner speziellen Stromform am Patienten erzielbare transkutane Behandlung ermöglicht bei Herpes-Formen ohne Schleimhautbeteiligung bereits am fünften bis siebenten Tag der Therapie ein Abtrocknen der sezernierenden Hautstellen und am zehnten bis vierzehnten Tag ein Abheilen der Effloreszenzen, wobei immunbiologische Prozesse eine führende Rolle spielen dürften. Auch bei Herpes-Formen mit Schleimhautbeteiligung ist eine deutliche Beschleunigung des Heilungsprozesses gegenüber der herkömmlichen Therapie zu erkennen. Bei Herpes-zoster mit Beteiligung des Nervus ophthalmicus wurde beobachtet, daß nach Anwendung des Therapiegerätes das Sehvermögen des befallenen Auges erhalten blieb. Nach den derzeitigen Erkenntnissen und Erfahrungen treten nach Behandlung mit dem erfindungsgemäßen Gerät keine oder nur selten Rezidive auf. Allenfalls auftretende Rezidive sind genau wie die Ersterkrankung zu behandeln, wobei zu erwarten ist, daß die Krankheitsdauer des zweiten Ausbruches wesentlich kürzer sein wird.

Die Stromstärke (mA) soll bei der Behandlung so eingestellt werden, bzw. vom Patienten selbst so vorgenommen
werden, daß er ein deutliches, aber nicht schmerzhaftes
Pulsieren an der Haut spürt. Nach ca. fünf Minuten ist es
meist notwendig, die Intensität zu erhöhen, um Gewöhnungsprozessen entgegenzuwirken und die ursprüngliche Ausgangsposition zu erreichen.

Bei der Behandlung von Herpes im Bereich des Gesichtes und
des Schädels wird beispielsweise der Nervus trigeminus mit
seinen drei Ästen entsprechend der erkrankten Gesichtshälfte
elektrisch behandelt. Dabei ist es nötig, je nach Befallsbereich des Herpes, das entsprechende Segment entweder einzeln
oder mehrfach zu behandeln, wobei die Anode als einzelne am
Ganglion gasseri plaziert wird. Die Kathoden hingegen werden an möglichst weit von der Anode entfernter Stelle des
erkrankten Dermatoms plaziert. Bei Befall mehrerer Dermatome der gleichen Gesichtshälfte können auch nur eine Anode
und mehrere Kathoden verwendet werden.

Bei einem Herpes-zoster, der sich über zwei oder drei Intercostalräume ausgebreitet hat, können entsprechend viele
Anoden plaziert werden, die Kathode hingegen wird gegenüber
- also am Bauch - homolateral angebracht.

Das erfindungsgemäße Therapiegerät kann sowohl beim akuten
Ausbruch mit oder ohne Effloreszenzen als auch für die Behandlung postherpetischer Schmerzen angewandt werden. Die
Behandlung wird so lange durchgeführt, bis die Effloreszenzen abgeheilt bzw. die Schmerzen sistiert bzw. signifikant
zurückgegangen sind. In vielen Fällen wird es notwendig
sein, der Patienten bei Beginn der Behandlung Analgetika
zu verabreichen, dies jedoch nur so lange, bis die Analgesie
durch die Behandlung ihre volle Wirkung erreicht hat.

Bei eventuellen Rezidiven sollte so bald wie möglich mit
der Behandlung begonnen werden, wobei empfohlen wird, im Abstand der zu erwartenden Rezidive die Behandlung beispielsweise an vierzehn aufeinanderfolgenden Tagen anzusetzen,
auch wenn zu diesem Zeitpunkt kein Ausbruch (Schmerz und/
oder Effloreszenzen) beobachtet wird.

Bei der Behandlung von Herpes-genitalis und Herpes-progenitalis, wird die Anode paravertebral transkutan entweder
rechts oder links am zweiten sakralen Segment plaziert.
Sollten sichtbare Effloreszenzen rund um die Genitalien
vorhanden sein, ist es notwendig, die entsprechenden Dermatome miteinzubeziehen.

Die Behandlung sollte grundsätzlich mit verschieden großen
Elektroden durchgeführt werden, wobei die Anode relativ
klein ist, um aufgrund der Feldliniendichte mit genügend
Strom an den Nerv heranzukommen. Dagegen muß die Kathode
relativ groß sein, um den kathodalen Reiz zu verteilen, damit der Patient weder Sensationen noch unangenehme Reize
spürt.

Als Elektroden können herkömmliche Kunststoff- oder Silikon-
Elektroden, die auch aus der Reizableitung z.B. bei EKG bekannt sind, verwendet werden. Dabei werden die Elektroden
z.B. mit einem leitenden Naturharz (Caraya) in der entsprechenden Größe belegt. Man fixiert diese nach Befeuchten mit
Wasser an der Haut. Die Elektroden können auch mit Elektrodengel dünn bestrichen, an der Haut angelegt und mit einem
Heftpflaster od. dgl. fixiert werden. Für die Behandlung im
Bereich des Kopfes werden Elektroden mit runder Kontaktfläche verwendet, wobei die Anode einen Durchmesser von etwa
13 mm aufweist und die Kathode etwa 29 mm mißt. Die am Kopf
als Kathode verwendete Elektrode findet am Körper als Anode
Verwendung. Die Kathode ist in diesem Fall um den kathodalen
Reiz großflächig zu verteilen, z.B. als Rechteck mit den Abmessungen 120 x 70 mm ausgebildet.

Von Vorteil bei der Behandlung ist es, wenn diese 10 bis
40 Minuten dauert. Als besonders günstig hat sich eine Behandlungsdauer von 20 Minuten herausgestellt.

Eine weitere Verbesserung wurde festgestellt, wenn die Behandlung in Abständen von etwa 5 Stunden wiederholt wird und wenn dieser Behandlungsrhythmus zumindest an fünf aufeinanderfolgenden Tagen aufrecht gehalten wird.

Als Beispiel sei hier die Behandlung bei generalisiertem Herpes angegeben. Es kann beobachtet werden, daß der Ausbruch bzw. die sichtbaren Effloreszenzen immer mit einem lokalen Herpes beginnen und sich erst dann ausweiten. Es wird dann der ursprüngliche lokale Herpes behandelt (z.B. bei Herpes-zoster intercostalis werden die Elektroden entsprechend der Dermatome paravertebral plaziert). Die Behandlung erfolgt dreimal täglich je 20 Minuten im Abstand von fünf Stunden und so lange, bis die Effloreszenzen, Schmerzen etc. abklingen.

Bei Patienten mit Leukämie kann es bekanntlich zu Ausbrüchen von Herpes nach Marktransplantationen kommen. Bei diesen Patienten, bei welchen die Körperabwehr nach Transplantation bewußt pharmakologisch herabgesetzt wurde, wird empfohlen, sofort nach der Transplantation prophylaktisch mit der Herpes-Behandlung nach der Erfindung zu beginnen.

Um eine besonders effektvolle, rasch wirksame Behandlung zu erzielen, sollte der Patient während der Dauer der Behandlung von Streß jeglicher Art soweit wie möglich ferngehalten werden.

Zur Stromversorgung des erfindungsgemäßen Gerätes reichen z.B. 8 NiCd-Akkus mit je 1,2 Volt und 600 mA. Über ein Tastenfeld kann der Behandlungsstrom stufenweise vergrößert bzw. verkleinert werden. Die Einheit zur Konstantstromerzeugung bildet und liefert, gesteuert vom Mikroprozessor, die zur Behandlung notwendigen Stromimpulse mit der vorher über das Tastenfeld eingegebenen Stromstärke.

Nach einer besonders vorteilhaften Ausgestaltung der Erfindung weist das Therapiegerät eine Einrichtung zur Strommessung mit einer Peak-hold-Schaltung zur Messung von Stromspitzen einzelner Stromimpulse auf, wobei die Einrichtung ein Steuersignal erzeugt, welches ggf. unter Zwischenschaltung eines Mikroprozessors in der Einheit zur Konstantstromerzeugung zur Stromabschaltung führt. Mit der erfindungsgemäßen Einrichtung ist es möglich, Stromspitzen einzelner Stromimpulse zu messen und das Gerät bei Überschreitung vorgebbarer Pegel automatisch abzuschalten.Diese Schaltung dient vor allem für die Sicherheit des Patienten und schützt ihn vor unangenehmen Stromstößen, welche beispielsweise durch statische Aufladung oder durch vorhandene Potentialdifferenzen zwischen dem erfindungsgemäßen Gerät und anderen mit dem Patienten in Berührung kommenden Gegenständen oder zur Behandlung notwendigen zusätzlichen Geräten, herrühren können.

Von Vorteil ist es, wenn erfindungsgemäß das Steuersignal der Einrichtung zur Strommessung ggf. unter Zwischenschaltung des Mikroprozessors einer vorzugsweise akustischen Anzeige- und Warneinrichtung zur Anzeige des Behandlungsendes oder zur Fehlermeldung zuführbar ist. Diese Anzeigeeinrichtung, beispielsweise in Form eines im Gerät integrierten Piezosummers oder eines kleinen Lautsprechers dient vor allem zur Anzeige des Endes einer 20minütigen Behandlung. Gleichzeitig können von dieser Einrichtung auch Warnfunktionen übernommen werden, beispielsweise das Anzeigen einer Stromunterbrechung durch schlechten Elektrodenkontakt oder auch die Unterspannung der Stromversorgung des Gerätes. Es ist natürlich auch möglich, optische Anzeige- und Warneinrichtungen vorzusehen, beispielsweise blinkende Leuchtdioden.

Weiters kann bei einem Gerät nach der Erfindung vorgesehen sein, daß eine Ladezustandskontrolle vorhanden ist, welche bei Unterspannung ein Signal zur Abschaltung des Gerätes erzeugt. Dies stellt eine weitere Sicherheitseinrichtung dar, die ggf. auch mit dem Mikroprozessor verbunden sein kann, wobei bei Unterbrechung einer Behandlung, bedingt durch Unterspannung, trotzdem die tatsächlich aufgewendete Behandlungszeit am Display des Behandlungsgerätes aufscheint.

Besonders vorteilhaft ist es beim erfindungsgemäßen Therapiegerät, wenn die Frequenz der Impulse 30 Hz und deren
Impulsdauer 0,2 ms beträgt. Frequenzen unter 5 Hz können
unangenehm spürbar sein (elektrische Stöße). Es ist zu beachten, daß durch Frequenzen über 180 Hz bereits motorische
Nerven blockiert werden könnten.

In einer vorteilhaften Ausgestaltung der Erfindung ist
vorgesehen, daß eine Speichereinheit zur Speicherung der
Impulsdaten vorzugsweise der Frequenz und der Impulsdauer
vorgesehen ist. Dabei kann vorzugsweise ein ROM Verwendung
finden, welches alle für die Impulserzeugung notwendigen
Daten, wie Frequenz, Impulsdauer und Impulsform in den Mikroprozessor einliest.

Schließlich ist es auch möglich, einen zusätzlichen Speicher mit dem Mikroprozessor zu verbinden, welcher Behandlungsdaten des Patienten, vorzugsweise die Anzahl und die
Dauer der Behandlungen, speichert. Damit können vor jeder
Behandlung spezifische Daten des jeweiligen Patienten abgerufen werden, womit der gewünschte Behandlungsrhythmus besser eingehalten werden kann.

Die Erfindung wird im folgenden anhand von Zeichnungen
näher erläutert. Es zeigen:

Figur 1 ein erfindungsgemäßes Therapiegerät zur Behandlung
von verschiedenen Formen von Herpes,

Figuren 2 bis 5 Elektrodenplazierungen für die Behandlung
von Herpes und in

Figur 6 ein Diagramm der Stromimpulse.

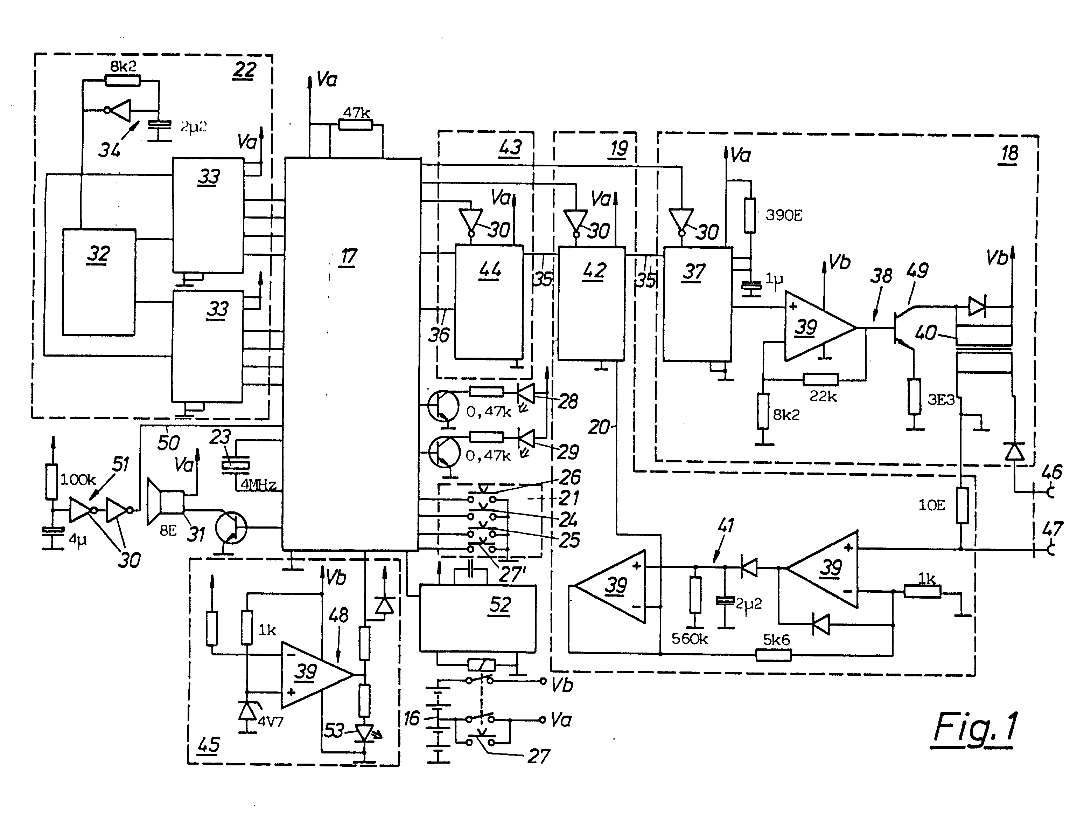

In Fig. 1 sind zur besseren Übersicht elektronische Bauteilgruppen mit strichlierten Linien zu Blöcken zusammengefaßt.

Zentraler Bauteil ist der Mikroprozessor 17, der zur Steuerung des gesamten Geräts dient und zwar zur Impulssteuerung, Einlesen der Tastenbefehle, welche über das Tastenfeld 21 einzugeben sind und zur Ansteuerung der Anzeigeeinheit 22. Die Stromversorgung 16 des Gerätes kann sowohl durch einen Batteriensatz als durch ein Netzgerät realisiert werden. Vom Mikroprozessor 17 wird eine Einheit 18 zur Konstantstromerzeugung angesteuert, welche schließlich die für die Behandlung benötigten Stromimpulse über die Elektroden an den Patienten abgibt. Eine Einrichtung 19 dient ausgangsseitig zur Strommessung, wobei die gemessenen Werte digitalisiert und über eine Rückkopplung 20 dem Mikroprozessor 17 zugeführt werden. Das Gerät weist auch eine Speichereinheit 43 und eine Ladezustandskontrolle 45 auf. Nach dem Einschalten des Gerätes mittels Tippschalter 27 liegt an den Versorgungsleitungen der einzelnen Bauteile die Versorgungsspannung von $V_a$ = 9,6V bzw. $V_b$ = 4,8V an. Der Mikroprozessor 17, beispielsweise ein R65 01Q von Rockwell, wird über seine Versorgungsleitung gespeist und mit einer Taktfrequenz (Quarz 23) von 4 MHz betrieben. Über die Tasten 24 bzw. 25 des Tastenfeldes 21 kann die Stromstärke des Behandlungsstromes vergrößert bzw. verkleinert werden. Die Taste 26 dient zum Umschalten der Anzeigeeinheit zur Anzeige der Behandlungsdauer, ein Tippschalter 27' zum Abschalten des Gerätes. Die Leuchtdioden 28, 29 zeigen den Betriebszustand der Anzeigeeinheit an (Strommessung oder Zeitmessung). Die Schaltung 51 (Power-up-Reset) am Reset-Eingang 50 des Mikroprozessors 17 sorgt dafür, daß das Gerät erst dann zu arbeiten beginnt, wenn der Mikroprozessor seine vorgegebene Taktfrequenz erreicht hat. Bei den mit 30 bezeichneten Bauteilen der Schaltung handelt es sich um digitale Inverter.

Das Gerät verfügt auch über eine akustische Anzeige- und Warneinrichtung 31, welche verschiedenste Funktionen erfüllt. So können beispielsweise der Ablauf der Behandlungszeit, die Unterbrechung des Stromflusses, durch Herunterfallen von Elektroden etc. oder interne Fehler des Gerätes akustisch gemeldet werden. Wenn kein Strom über die Elektroden fließt, spricht die Warneinrichtung an und der Strom wird abgeschaltet, wobei das Gerät eingeschaltet bleibt. Die Anzeigeeinheit 22 des Gerätes umfaßt ein LC-Display 32, z.B. LD-H 7980 AZ von Epson, welches von zwei LCD-Treibern 33 (74 HC 4543 National ) angesteuert wird. Hier erfolgt auch die Dekodierung von BCD auf 7-Segment. Ein Oszillator 34 sorgt für die Erzeugung einer Rechteckspannung von ca. 60 Hz zur Versorgung des LC-Displays 32.

Über die digitale Datenleitung 35 ist ein Digital-Analogkonverter 37 (ZN 428 von Ferranti) mit dem Mikroprozessor 17 verbunden. Dieser dient zur Umwandlung der digitalen Stromvorgabe in analoge Form und zur Ansteuerung der Konstantstrom-Ausgangsstufe 38 mit einem Operationsverstärker 39, einem Leistungstransistor 49 (Darlington MPSU 45) und einem Impuls-Transformator 40, welcher sekundärseitig mit den Elektroden 46, 47 verbunden und in der Lage ist, steile Impulsflanken möglichst unverzerrt zu übertragen.

Die Einheit zur Strommessung 19, weist eine Peak-hold-Schaltung 41 zur Erkennung von eventuell auftretenden Stromspitzen und zur kontinuierlichen Strommessung auf, sowie einen über die digitale Datenleitung 35 mit dem Mikroprozessor 17 verbundenen Analog-Digitalkonverter 42, beispielsweise ADC 0804 von National, zur Umwandlung des analogen Meßwertes in digitale Form und Weiterleitung zum Mikroprozessor. Bei den mit 39 bezeichneten Bauteilen der Schaltung 41 handelt es sich um Operationsverstärker (LM 2902 von National).

Das Gerät verfügt auch über eine Speichereinheit 43, die einen Programmspeicher 44, beispielsweise ein MBM 27C32 A von Fujitsu, aufweist, welcher über die digitale Datenleitung 35 und die Adressenleitung 36 mit dem Mikroprozessor 17 verbunden ist. Dieses ROM enthält alle Parameter für die Impulsregelung, wie Frequenz, Impulsdauer, etc.

Mit einer weiteren Speichereinheit wäre es auch möglich, patientenspezifische Behandlungsparameter zu speichern, was zu einer Vereinfachung bei Mehrfachbehandlungen führt.

Weiters verfügt das Gerät über eine Ladezustandskontrolle 45, welche eine Komparatorschaltung 48 zur Erkennung einer Unterspannung aufweist. Bei Unterspannung erfolgt die Anzeige über die Leuchtdiode 53. Die Ladezustandskontrolle ist mit dem Mikroprozessor 17 verbunden und löst über diesen bei Unterspannung ein akustisches Alarmsignal aus, wonach das Gerät automatisch abschaltet.

Ein Bauteil 52 (74HC123 von National) mit einer sogenannten Watchdog-Schaltung, stellt eine Sicherheitseinrichtung dar, welche den Mikroprozessor 17 überwacht und kontrolliert. Eine Unterbrechung oder Abweichung im Programmablauf führt zur Abschaltung des Gerätes.

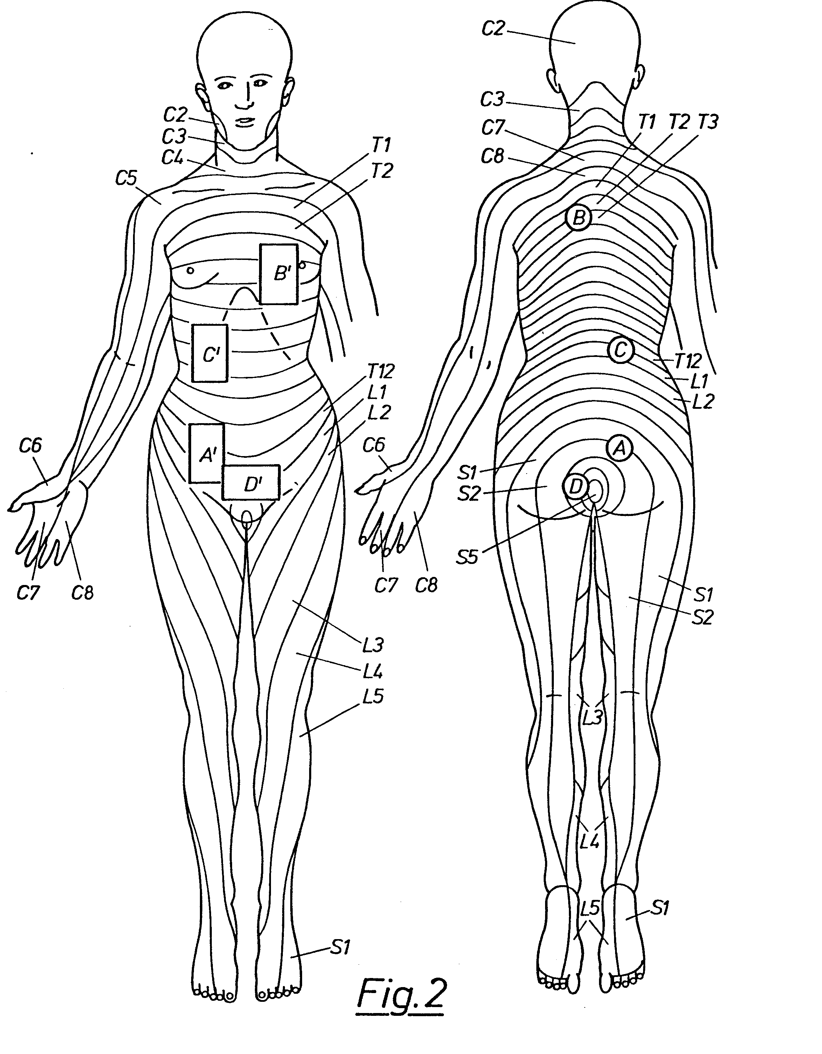

In Fig. 2 sind mit C2 bis C8 die cervikalen Segmente bezeichnet, mit T1 bis T12 die thorakalen, mit L1 bis L5 die lumbalen und mit S1 bis S5 die sakralen. Es seien nun einige typische Elektrodenplazierungen bei Herpes-Befall angegeben:

Herpes-genitalis: Anode A auf Segment S2 rechts oder links, Kathode A' gegenüber am Bauch, homolateral.

Herpes-zoster: Anode B auf Segment T3 rechts oder links, Kathode B' gegenüber am Thorax, homolateral.

Herpes-progenitalis: Anode C auf Segment L1 rechts oder links, Kathode C' gegenüber am Thorax bzw. Bauch, homolateral.

Herpes-analis: Anode D am Segment S5 abwechselnd rechts und links, Kathode D' gegenüber, quer an der Symphyse, median.

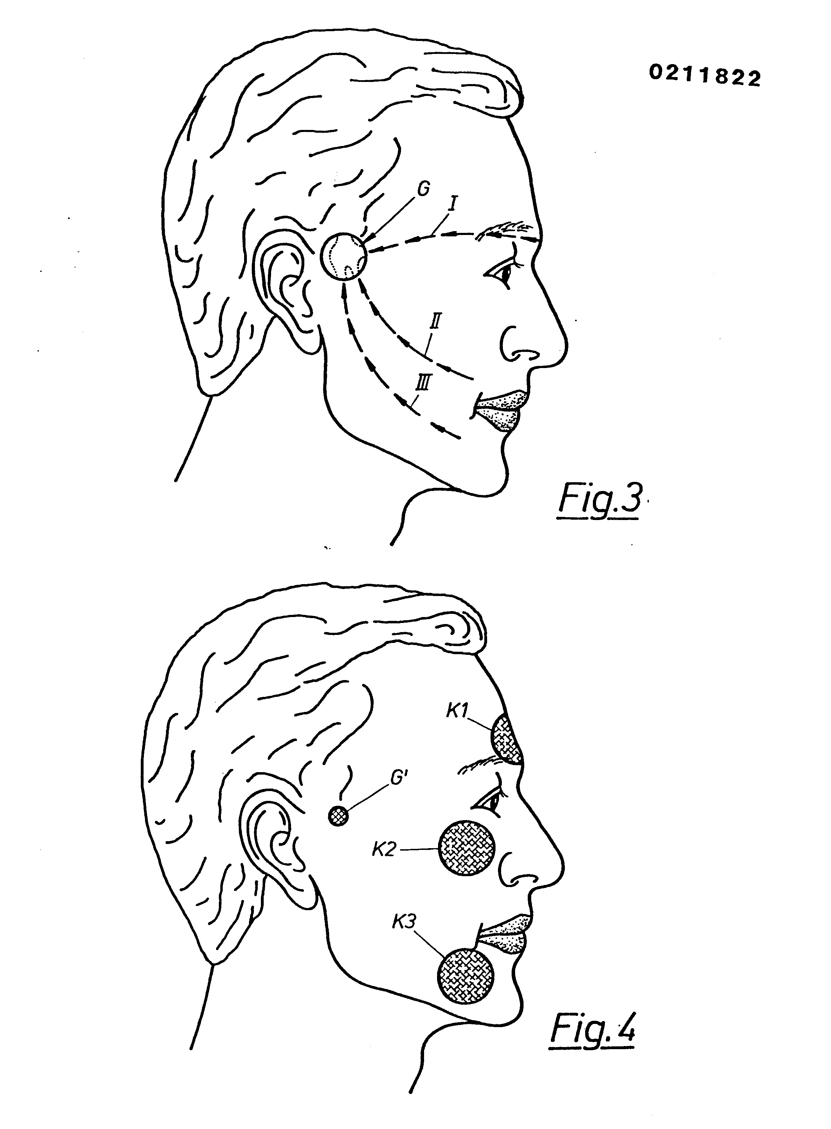

In der Fig. 3 ist die Stelle an der Kopfoberfläche unter der das Ganglion trigeminale (gasseri) liegt, mit G bezeichnet. Von diesem Ganglion ziehen die drei Äste des Nervus trigeminus aus. Es sind dies der Nervus ophthalmicus I, der Nervus maxillaris II und der Nervus mandibularis III.

Bei Befall eines oder mehrerer dieser Nervenäste mit Herpes werden die Elektroden laut Fig. 4 plaziert. Dabei kommt die Anode G', deren Kontaktfläche einen Durchmesser von etwa 13 mm aufweist, über dem Ganglion gasseri zu liegen. Die Kathoden K1 bis K3, die einen Durchmesser von etwa 29 mm aufweisen, liegen an den in Fig. 4 bezeichneten Stellen.

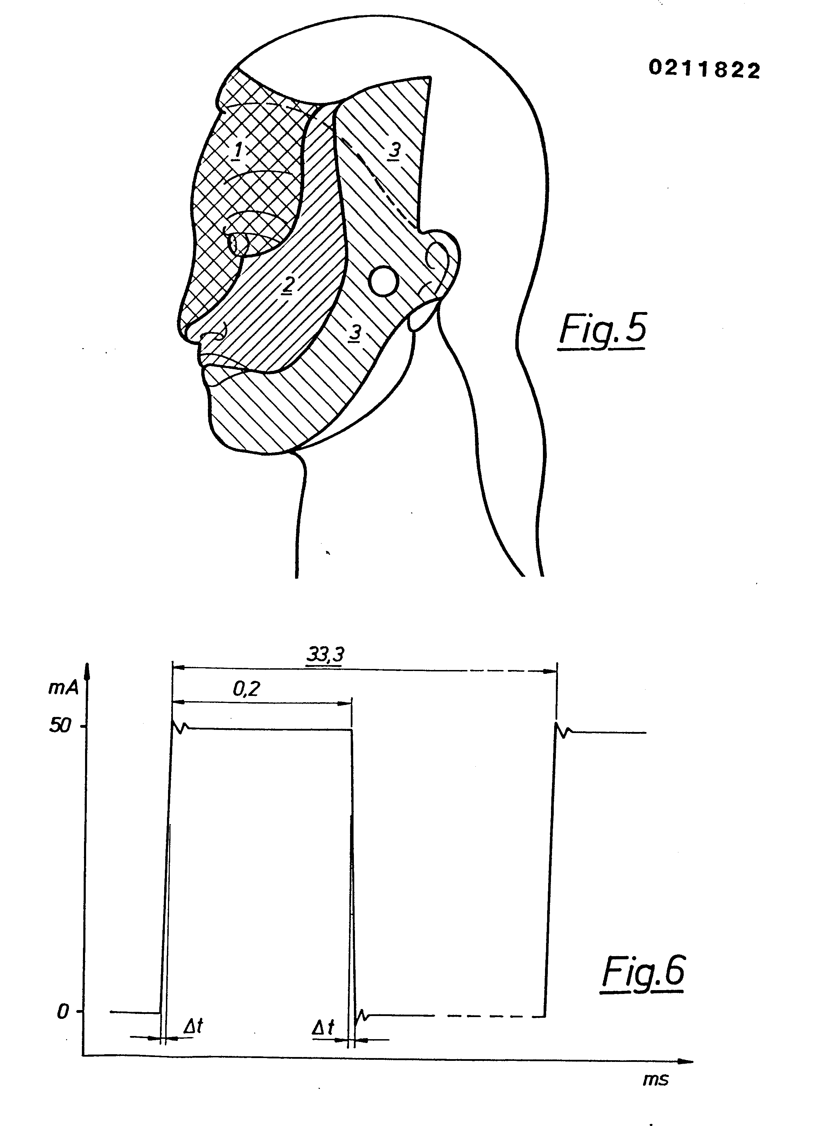

In Fig. 5 sind die von den drei Ästen des Nervus trigeminus versorgten Dermatome mit den Ziffern 1 bis 3 bezeichnet. So ist beispielsweise bei Herpes-labialis je nach Befall an Ober- oder Unterlippe das entsprechende Dermatom (zweites und/oder drittes) zu behandeln. Die Anode G' ist am Ganglion gasseri und die Kathode oder die Kathoden jeweils an den bezeichneten Elektrodenlagen K2 und/oder K3 (Fig. 4) zu plazieren. Sollte der Befall auch das erste Segment einschließen, wird es nötig sein, eine weitere Kathode K1 vor der jeweiligen Augenbraue zu fixieren. Es ist im Gesicht gut möglich, mit einer Anode und drei Kathoden zu behandeln. Die gleichen Dermatome werden auch bei Befall der Mundschleimhäute in der Mundhöhle behandelt.

Über die am erkrankten Dermatom anzubringenden Elektroden 46, 47 werden monopolare annähernd rechteckförmige Stromimpulse der in Fig. 6 dargestellten Impulsform an den Patienten abgegeben. Dabei beträgt die Impulsdauer 0,2 msec und die Stromstärke beispielsweise 50 mA. Letztere kann von 0 bis 60 mA variiert werden und wird in Abhängigkeit vom Hautwiderstand des Patienten geregelt und konstant gehalten. Die Frequenz der Impulse beträgt 30 Hz. Es ist darauf zu achten, möglichst steile Impulsflanken (kleines $\Delta t$) mit geringen Über- bzw. Unterschwingungen zu erreichen.

Patentansprüche:

1. Elektromedizinisches Therapiegerät, mit einer Einheit zur Konstantstromerzeugung, welches eine Folge von monopolaren Stromimpulsen von annähernd Rechteckform erzeugt, die über zwei an der Sekundärwicklung eines Impulstransformators angeschlossene Elektroden der Hautoberfläche eines Patienten zuführbar sind, d a d u r c h   g e k e n n z e i c h n e t,   daß die Frequenz der Stromimpulse zweischen  25 und 35 Hz liegt und die Impulsdauer zwischen 0,1 und 0,3 ms beträgt, sowie daß die Stromstärke abhängig von den über eine Eingabeeinrichtung (21) vorgebbaren Werten kleiner gleich 60 mA beträgt, wodurch Herpesviren in organischem Gewebe inaktiviert werden.

2. Elektromedizinisches Therapiegerät nach Anspruch 1, gekennzeichnet durch eine Einrichtung (19) zur Strommessung, mit einer Peak-hold-Schaltung (4) zur Messung von Stromspitzen einzelner Stromimpulse, wobei die Einrichtung (19) ein Steuersignal (I) erzeugt, welches ggf. unter Zwischenschaltung eines Mikroprozessors (17) in der Einheit (18) zur Konstantstromerzeugung zur Stromabschaltung führt.

3. Elektromedizinisches Therapiegerät nach Anspruch 2, dadurch gekennzeichnet, daß das Steuersignal (I) der Einrichtung (19) zur Strommessung ggf. unter Zwischenschaltung des Mikroprozessors (17) einer vorzugsweise akustischen Anzeige- und Warneinrichtung (31) zur Anzeige des Behandlungsendes oder zur Fehlermeldung zuführbar ist.

4. Elektromedizinisches Therapiegerät nach einem der Ansprüche 1 bis 3, dadurch gekennzeichnet, daß eine Ladzustandskontrolle (45) vorhanden ist, welche bei Unterspannung ein Signal zur Abschaltung des Gerätes erzeugt.

5. Elektromedizinisches Therapiegerät nach einem der An-

- 15 -

**0211822**

sprüche 1 bis 4, dadurch gekennzeichnet, daß die Frequenz der Impulse  30 Hz und deren Impulsdauer 0,2 ms beträgt.

6. Elektromedizinisches Therapiegerät nach einem der Ansprüche 1 bis 5, dadurch gekennzeichnet, daß eine Speichereinheit (44) zur Speicherung der Impulsdaten vorzugsweise der Frequenz und der Impulsdauer vorgesehen ist.

1986 06 19
Kr/Lu/Fr

Fig.1

0211822

_Fig.2_

0211822

_Fig.3_

_Fig.4_

0211822

Fig.5

Fig.6